# EUROPÄISCHE PATENTSCHRIFT

(11) **EP 1 373 279 B1**
(45) Veröffentlichungstag und Bekanntmachung des Hinweises auf die Patenterteilung: **17.05.2006**
(21) Anmeldenummer: 02735144.4
(22) Anmeldetag: 19.03.2002
(51) Int. Cl.: C07F 9/50, C07F 9/52, C07F 9/48, C07C 45/50, B01J 31/24

(54) **VERFAHREN ZUR HERSTELLUNG VON DIPHOSPHINEN UND DEREN VERWENDUNG**
METHOD FOR PRODUCING DIPHOSPHINES AND THE USE THEREOF
PROCEDE DE PRODUCTION DE DIPHOSPHINES ET LEUR UTILISATION

(30) Priorität: 26.03.2001 DE 10114868
(43) Veröffentlichungstag der Anmeldung: 02.01.2004
(73) Patentinhaber: Oxeno Olefinchemie GmbH, 45772 Marl (DE)
(72) Erfinder: JACKSTELL, Ralf, 27472 Cuxhaven (DE); KLEIN, Holger, 18069 Rockstock (DE); BELLER, Matthias, 18211 Nienhagen (DE); WIESE, Klaus-Diether, 45721 Haltern (DE); BORGMANN, Cornelia, 45657 Recklinghausen (DE)
(86) Internationale Anmeldenummer: PCT/EP2002/002989
(87) Internationale Veröffentlichungsnummer: WO 2002/076996

(56) Entgegenhaltungen:
- EP-A- 0 653 432
- KLEIN H ET AL: "Highly selective catalyst systems for the hydroformylation of internal olefins to internal aldehydes" ANGEWANDTE CHEMIE, INTERNATIONAL EDITION, Bd. 40, Nr. 18, 17. September 2001 (2001-09-17), Seiten 3408-3411, XP002203112
- GILLIE A ET AL: "Mechanisms of 1,1-Reductive Elimination from Palladium" JOURNAL OF THE AMERICAN CHEMICAL SOCIETY, Bd. 102, Nr. 15, 1980, Seiten 4933-4941, XP002203113
- DANI P ET AL: "Replacement of a Cyclometalated Terdentate Diamino Ligand by a Phosphorus Analogue. Isolation and Crystallographic Characterisation of an Intermediate in Aryl C-H Bond Activation in Models of Dendrimer-Bound Organometallic Catalysts" JOURNAL OF THE AMERICAN CHEMICAL SOCIETY, Bd. 119, Nr. 46, 1997, Seiten 11317-11318, XP002203114
- YAMAMOTO Y ET AL: "Preparation and characterisation of palladium and platinum complexes bearing 1,8-bis[(diphenylphosphino)methyl]naphthal ene" JOURNAL OF THE CHEMICAL SOCIETY, DALTON TRANSACTIONS, 2001, Seiten 1773-1781, XP002203115
- GOERTZ W ET AL: "Asymmetric Nickel-Catalysed Hydrocyanation of Vinylarenes by Applying Homochiral Xantphos Ligands" CHEMISTRY, A EUROPEAN JOURNAL, Bd. 7, Nr. 8, 2001, Seiten 1614-1618, XP002203116
- GOERTZ W ET AL: "Electronic effects in the nickel-catalysed hydrocyanation of styrene applying chelating phosphorus ligands with large bite angles" JOURNAL OF THE CHEMICAL SOCIETY, DALTON TRANSACTIONS, 1998, Seiten 2981-2988, XP002195283
- BENTRUDE W G ET AL: "Free-radical chemistry of organophosphorus compound. IV. Polar, bond strength and resonance effects on phosphoranyl radical formation" JOURNAL OF THE AMERICAN CHEMICAL SOCIETY, Bd. 95, Nr. 11, 30. Mai 1973 (1973-05-30), Seiten 3625-3635, XP002203117
- EVLETH E M ET AL: "P-PHENYLENEDIPHOSPHINE AND RELATED COMPOUNDS" JOURNAL OF ORGANIC CHEMISTRY, AMERICAN CHEMICAL SOCIETY. EASTON, US, Bd. 27, Juni 1962 (1962-06), Seiten 2192-2197, XP001021629 ISSN: 0022-3263
- MARINETTI A ET AL: "NEW CHIRAL DICHLOROPHOSPHINES AND THEIR USE IN THE SYNTHESIS OF PHOSPHETANE OXIDES AND PHOSPHINIC CHLORIDES" JOURNAL OF ORGANIC CHEMISTRY, AMERICAN CHEMICAL SOCIETY. EASTON, US, Bd. 62, Nr. 2, 24. Januar 1997 (1997-01-24), Seiten 297-301, XP000640625 ISSN: 0022-3263
- REETZ M T ET AL: "New non-C2-symmetric phosphine-phosphonites as ligands in asymmetric metal catalysis" TETRAHEDRON: ASYMMETRY, Bd. 10, Nr. 11, 4. Juni 1999 (1999-06-04), Seiten 2129-2137, XP004174097 ISSN: 0957-4166
- VAN DOORN J A ET AL: "Reductive cleavage of the carbon-phosphorus bond with alkali metals. III. Reactions of arylalkylphosphines" RECUEIL DES TRAVAUX CHIMIQUES DES PAYS-BAS, Bd. 111, Nr. 4, 1992, Seiten 170-177, XP001084949

## Beschreibung

Die vorliegende Erfindung betrifft ein verbessertes Verfahren zur Herstellung von Diphosphinen wie Bis(diarylphosphinylmethyl)arenen und deren Verwendung.

Phosphine finden vielseitige technische Anwendungen. Sie besitzen industrielle Bedeutung als Antioxidantien, Metallextraktionsmittel, Flammschutzimprägniermittel, Stabilisierungsmittel für Olefine, Ausgangsmaterialien für Wittig-Reagenzien und insbesondere als Liganden für Übergangsmetall-Katalysatoren.

Eine Übersicht zu den wichtigen Homogenkatalysatoren mit Phosphinliganden findet man beispielsweise in B. Cornils, W. A. Herrmann, Applied Homogeneous Catalysis with Organometallic Compounds Vol. 1 & 2, VCH, Weinheim, 1996. Innerhalb der Gruppe der Phosphine sind chelatisierende Phosphine, insbesondere Diphosphine, als Liganden für Metallkomplexe von besonderer Bedeutung. Dies beruht darauf, dass Diphosphine stabilere Komplexe mit dem entsprechenden Zentralmetallatom eingehen und dadurch dessen katalytische Eigenschaften nachhaltiger beeinflussen. Aus der Gruppe der bekannten chelatisierenden Diphosphine sind Bis-(diarylphosphinyl-alkyl)-1,1'-binaphthyle als Bestandteile von Carbonylierungskatalysatoren und Telomerisationskatalysatoren von industriellem Interesse. EP 0 653 432, EP 0 673 944 und JP 7939059 beschreiben die Herstellung von Bis-(diarylphosphinyl-alkyl)-1,1'-binaphthylen durch Umsetzung von 2,2'-Dimethylbinaphthylen mit einem Bromierungsmittel zu 2,2'-Bis(brommethyl)-1,1'-binaphthyl, anschließende Reaktion mit einem Diphenylphosphinigsäurealkylesters und anschließende Reduktion des entstandenen Bisphosphinoxids mit einem Organyldichlorsilan. Dieses Verfahren hat mehrere grundsätzliche Nachteile: um die Phosphorgruppen in die Zielmoleküle einzuführen, muss zunächst bromiert werden, dann werden die Bromatome gegen Phosphorgruppen ausgetauscht. Dies bedingt den Einsatz von teuren und giftigen Bromierungsreagenzien sowie den Anfall von überstöchiometrischen Mengen an Bromidabfällen. Die Bromierung der Ausgangsverbindungen führt generell zu Produktgemischen, dessen Handhabung problematisch ist, da die entstehenden Arylalkylbromide tränenreizende und gesundheitsgefährdende Stoffe darstellen. Darüber hinaus führt die Umsetzung mit Diphenylphosphinigsäurealkylestern zum Phosphinoxid, das in einer weiteren Stufe reduziert werden muss. Ausgehend von 2,2'-Dimethylbinaphthylen sind somit 4 Stufen notwendig um die gewünschten Liganden zu erhalten.

Ähnliche Synthesen oder Teilsynthesen mit den gleichen und zum Teil weiteren Nachteilen sind auch in der Literatur beschrieben (M. E. Jung, et al. Tetrahedron Lett. 1988, 29, 6199; H. J. Bestmann et al. Chem. Ber. 1974, 2926; T. Hayashi et al. J. Am. Chem. Soc. 1988, 110, 8153).

In EP 0 704 449 werden unsymmetrisch substituierte Bis-(diarylphosphinyl-alkyl)-1,1'-binaphthyle aus 2,2'-Bis(brommethyl)-1,1'-binaphthyl über entsprechende Phosphoniumsalze herstellt. Dieses Verfahren weist die oben genannten Nachteile auf und ist für die Herstellung symmetrischer Bis-(diarylphosphinyl-alkyl)-1,1'-binaphthyle nicht vorteilhaft, da die Phosphorgruppen hier in zwei Stufen in das Zielmolekül eingeführt werden müssen und große Mengen an Salzabfällen anfallen.

In EP 0 653 432 wird ein Verfahren zur Herstellung von Bis-(diaryl-alkyl)-phosphinen bei dem ein Bis-diaryl-dialkyl-phosphinoxid mit einem Organyldichlorsilan in Gegenwart eines tertiären Amins umgesetzt wird.

In Journal of the American Chemical Society, Bd. 102, Nr. 15, 1980, Seiten 4933 bis 4941, Journal of the American Chemical Society, Bd 119, Nr. 46, 1997, Seiten 11317 bis 11318 und Journal of the Chemical Society, Dalton Transactions, 2001, Seiten 1773 bis 1781 werden Diphosphine wie z. B. 1,8-Bis[(diphenylphosphino)methyl]naphthalin als Liganden beschrieben.

In Chemistry, a European Journal, Bd. 7, Nr. 8, 2001, S. 1614-1618, Journal of the Chemical Society, Dalton Transactions, 1998, S. 2981 bis 2988 und Journal of Organic Chemistry, Bd. 27. Juni 1962, S. 2192 - 2197 wird die Herstellung von Bis(bis-diethylamino)phosphinyl-Gruppen aufweisenden Verbindungen beschrieben.

In Journal of the American Chemical Society, Bd. 95, Nr. 11, 30. Mai 1973, S. 3625-3635, Journal of Organic Chemistry, Bd. 62, Nr. 2, 24. Januar 1997, S. 297-301 und Tetrahedron Asymmetry, Bd. 10, Nr. 11, 4. Juni 1999, S. 2129-2137 wird die Herstellung von Dichlorphosphinyl-Gruppen aufweisenden Verbindungen durch Umsetzung entsprechender Bis(bis-diethylamino)phosphinyl-Gruppen aufweisender Verbindungen mit HCl beschrieben.

In Recueil Des Travaux Chimique des Pays-Bas, Bd. 111, Nr. 4, 1992, S. 170 - 177 wird die Herstellung von Benzylphosphinen durch Reaktion von Benzyldichlorophosphinen mit einer aromatischen organometallischen Verbindung (ArLi oder ArMgX) beschrieben.

Aus den genannten Gründen bestand ein Bedarf, ein neues Verfahren zur Herstellung von Bis-(diarylphosphinyl-alkyl)-1,1'-binaphthylen zu entwickeln, das die vorstehend genannten Nachteile nicht aufweist, sich darüber hinaus einfach und sicher ausführen läßt und das gewünschte Wertprodukt in hoher Ausbeute und Reinheit zugänglich macht.

Diese Aufgabe wird gelöst durch ein Verfahren zur Herstellung von Diphosphinen der allgemeinen Formel I

R¹R²P-H₂C-Ar-CH₂-PR¹R² (I)

dadurch gekennzeichnet,
dass
a) Dimethylverbindungen der allgemeinen Formel II,

   H₃C-Ar-CH₃ (II)

   mit einer Base und einem N-substituierten Aminophosphorhalogenid zur Bis-(aminophosphinyl-methyl)-verbindung der allgemeinen Formel III

   R³R⁴P-H₂C-Ar-CH₂-PR³R⁴, (III)
b) die so erhaltenen Verbindungen der allgemeinen Formel III mit HCl zur Bis(dichlorphosphinyl-methyl)-Verbindung der allgemeinen Formel IV

   Cl₂P-H₂C-Ar-CH₂-PCl₂ (IV)
und c) die so erhaltenen Verbindungen der allgemeinen Formel IV mit metallorganischen Reagenzien zu den Zielverbindungen der allgemeinen Formel I umgesetzt werden, wobei R¹ und R² unabhängig voneinander für einen substituierten oder unsubstituierten aromatischen, heteroaromatischen oder aliphatischen Kohlenwasserstoffrest stehen, die eine kovalente Bindung zueinander aufweisen können und Ar für einen substituierten oder unsubstituierten aromatischen oder heteroaromatischen Kohlenwasserstoffrest und R³ und R⁴ unabhängig voneinander für einen N-subtituierten Aminoalkyl oder aminoarylrest stehen.

Das erfindungsgemäße Verfahren ist besonders interessant, da in der Vergangenheit die Herstellwege zu den Liganden der allgemeinen Formel I immer über Benzylhalogendzwischenstufen beschrieben wurden. Mit dem erfindungsgemäßen Verfahren können Diphosphine in guten Ausbeuten und hohen Reinheiten direkt aus den Verbindungen gemäß der allgemeinen Formel II erhalten werden.

Als Basen für die Umsetzung von Verbindungen der allgemeinen Formel II im Verfahrensschritt a) können beliebige Basen, die benzylische CH₃-Gruppen deprotonieren können, verwendet werden. Typische Beispiele für solche Basen sind Alkyl- oder Aryllithium-Verbindungen wie Butyllithium oder Methyllithium, Alkali- oder Erdalkalihydride wie Natrium- oder Kaliumhydrid, Alkali- oder Erdalkalimetalle wie Natrium oder Kalium in flüssigem Ammoniak, sowie Alkali- oder Erdalkalihydroxide oder Alkali- oder Erdalkalialkoxide wie beispielsweise Kalium-tert.butanolat.

Das Verfahren zur Herstellung von Diphosphinen gemäß der Erfindung kann durch das folgende Syntheseschema am Beispiel von 2,2'-Bis(diarylphosphüunethyl)-1,1'-binaphthylen wiedergegeben werden:

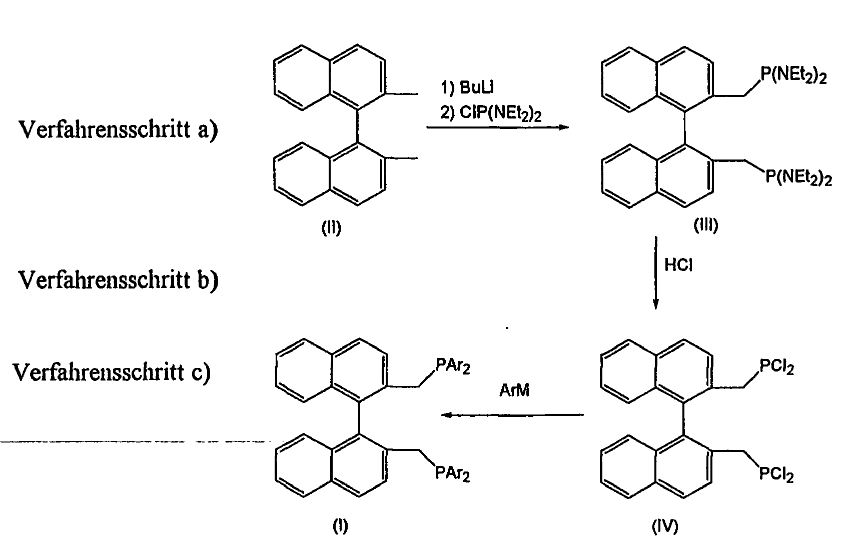

Als N-substituierte Aminophosphorhalogenide in Verfahrensschritt a) können Alkyl- oder Arylaminophosphorchloride, -bromide oder -iodide eingesetzt werden, wobei für Alkyl ein Kohlenwasserstoffrest aus der Gruppe Methyl-, Ethyl-, Propyl-, Isopropyl-, Butyl-, Isobutyl-, Hexyl-, 2-Ethylhexyl-, Octyl-, Isooctyl-, Nonyl- oder Isononyl-Rest und/oder diese Reste zueinander kovalente Verbindungen aufweisen und/oder für Alkylamino ein bis zu 6 C-Atomen enthaltender aliphatischer heterocyclischer Rest und/oder für Aryl ein bis zu 14 C-Atomen enthaltender aromatischer Rest stehen können, wobei diese Reste bis zu sieben Substituenten aus der Gruppe (C₁-C₈)-Alkyl, O-Alkyl-(C₁-C₈), O-Phenyl, Phenyl, Fluor, Chlor, CN, COOH, CHO, SO₃H, SO₂-Alkyl-(C₁-C₆), SO-Alkyl-(C₁-C₆), CF₃, COO-Alkyl-(C₁-C₈), COO-Phenyl, PO-Phenyl₂, POAlkyl₂-(C₁-C₄), PO₃H₂, PO(O-Alkyl-(C₁-C₆))₂, SO₃-Alkyl-(C₁-C₄) tragen können.

Als Alkylamino-Rest kann ein aliphatischer heterocyclischer Rest wie Piperidin oder Pyrrol und/oder für einen Aryl-Rest ein aromatischer Rest wie Phenyl-, Naphthyl- oder Benzylstehen. Bevorzugt finden Bis(dialkylamino)phosphorchloride Verwendung. Für alle Verbindungen gilt, dass die N-P-Bindung säurespaltbar sein muß.

### Nähere Beschreibung der allgemeinen Formel I

Die Reste R¹ und R² können jeweils gleich oder verschieden sein und für Aromaten mit bis zu 14 C-Atomen stehen, die bis zu acht Substituenten aus der Gruppe (C₁-C₈)-Alkyl, O-Alkyl-(C₁-C₈), O-Phenyl, Phenyl, Fluor, Chlor, CN, COOH, CHO, SO₃H, SO₂-Alkyl-(C₁-C₆), SO-Alkyl-(C₁-C₆), CF₃, COO-Alkyl-(C₁-C₈), COO-Phenyl, PO-Phenyl₂, POAlkyl₂-(C₁-C₄), PO₃H₂, PO(O-Alkyl-(C₁-C₆))₂, SO₃-Alkyl-(C₁-C₄) tragen können.

Weiterhin können die Reste R¹ und R² jeweils gleich oder verschieden sein und für Heteroaromaten mit einem fünf-, sechs- oder siebengliedrigen Ring mit Stickstoff-, Sauerstoff- oder Schwefelatomen im Ring, wobei an den Ring weitere aromatische, heteroaromatische und/oder aliphatische Ringe ankondensiert sein und die Ringe bis zu sieben Substituenten aus der Gruppe (C₁-C₈)-Alkyl, O-Alkyl-(C₁-C₈), O-Phenyl, Phenyl, Fluor, Chlor, CN, COOH, CHO, SO₃H, SO₂-Alkyl-(C₁-C₆), SO-Alkyl-(C₁-C₆), CF₃, COO Alkyl-(C₁-C₈), COO-Plienyl, PO-Phenyl₂, POAlkyl₂-(C₁-C₄), PO₃H₂, PO(O-Alkyl-(C₁-C₆))₂, SO₃-Alkyl-(C₁-C₄) tragen können, stehen.

Außerdem können die Kohlenwasserstoffreste R¹ und R² jeweils gleich oder verschieden sein und für bis zu 10 C-Atomen enthaltende cyclische oder acyclische Alkylreste stehen, die bis zu sieben Substituenten aus der Gruppe (C₁-C₈)-Alkyl, O-Alkyl-(C₁-C₈), O-Phenyl, Phenyl, Fluor, Chlor, CN, COOH, CHO, SO₃H, SO₂-Alkyl-(C₁-C₆), SO-Alkyl-(C₁-C₆), CF₃, COO-Alkyl-(C₁₋C₈), COO-Phenyl, PO-Phenyl₂, POAlkyl₂-(C₁-C₄), PO₃H₂, PO(O-Alkyl-(C₁-C₆))₂, SO₃-Alkyl-(C₁-C₄) tragen können.

Die Reste R¹ und R² können in diesen Fällen zusätzlich eine oder mehrere kovalente Bedingungen zu einander aufweisen und so z. B. ein Diphenyl-, Naphthyl- oder cyclisches System ausbilden.
Ar kann für ein Phenyl-, Naphthyl-, Anthracenyl- oder Phenanthrenylrest, eine 1,1'-Biphenyleinheit oder eine 1,1'-Binaphthyleinheit stehen, die jeweils bis zu acht weitere Substituenten aus der Gruppe (C₁-C₈)-Alkyl, O-Alkyl-(C₁-C₈), O-Phenyl, Phenyl, Fluor, Chlor, CN, COOH, CHO, SO₃H, SO₂-Alkyl-(C₁-C₆), SO-Alkyl-(C₁-C₆), CF₃, COO-Alkyl-(C₁-C₈), COO-Phenyl, PO-Phenyl₂, POAlkyl₂-(C₁-C₄), PO₃H₂, PO(O-Alkyl-(C₁-C₆))₂, SO₃-Alkyl-(C₁₋C₄) oder einen oder mehrere ankondensierte Phenylringe tragen können, stehen.

Alternativ kann Ar für Heteroaromaten mit einem fünf-, sechs- oder siebengliedrigen Ring mit Stickstoff-, Sauerstoff- oder Schwefelatomen im Ring, wobei an den Ring weitere aromatische, heteroaromatische und/oder aliphatische Ringe ankondensiert sein und die Ringe bis zu sechs Substituenten aus der Gruppe (C₁-C₈)-Alkyl, O-Alkyl-(C₁-C₈), O-Phenyl, Phenyl, Fluor, Chlor, CN, COOH, CHO, SO₃H, SO₂-Alkyl-(C₁-C₆), SO-Alkyl-(C₁-C₆), CF₃, COO-Alkyl-(C₁-C₈), COO-Phenyl, PO-Phenyl₂, POAlkyl₂-(C₁-C₄), PO₃H₂, PO(O-Alkyl-(C₁-C₆))₂, SO₃-Alkyl-(C₁₋C₄) tragen können, stehen.

Als metallorganische Reagenzien für die Umsetzung von Verbindungen der allgemeinen Formel IV zu den Zielverbindungen der allgemeinen Formel I im Verfahrensschritt c) finden Übergangsmetallarene, aliphatische oder aromatische Alkali- oder Erdalkaliverbindungen Verwendung. Besondere Verwendung finden aliphatische oder aromatische Grignardreagenzien, Alkyl- oder Arylzinkreagenzien, Alkyl- Aryllithiumreageuzien und/oder Arylkupferverbindungen. Die metallorganischen Reagenzien werden im allgemeinen vor der Umsetzung separat hergestellt, sie können jedoch auch bei der Reaktion in situ erzeugt werden.

Als Lösungsmittel finden bei den einzelnen Verfahrensschritten a), b) und c) des erfindungsgemäßen Verfahrens inerte organische Lösungsmittel Verwendung. Ohne Anspruch auf Vollständigkeit erheben zu wollen, seien hier beispielhaft polar aprotische Lösungsmittel, aliphatische Ether, aromatische oder aliphatische Kohlenwasserstoffe, aromatische und aliphatische Halogenkohlenwasserstoffe, Alkohole, Ester, Amide sowie deren Gemische genannt. Diese Lösungsmittel dürfen natürlich nicht mit den Reaktanden chemische Umsetzungen eingehen.

Die Verfahrensschritte a), b) und c) laufen vorzugsweise bei Temperaturen von -80 bis 200 °C ab; in den meisten Fällen hat es sich bewährt, bei Temperaturen von -30 bis 180 °C, besonders bevorzugt -20 bis 160 °C, zu arbeiten.

Der besondere Vorteil des erfindungsgemäßen Verfahrens liegt darin, dass keine toxischen Benzyl- und Alkylbromide hergestellt werden und dass keine Reduktion von Phosphinoxiden vorgenommen werden muss.

Aufgrund der Stabilität der Zwischenstufen gemäß der allgemeinen Formeln III und IV ist es möglich, diese Zwischenprodukte durch einfache Kristallisation aufzureinigen, so dass aufwendige Reinigungsverfahren wie Chromatographie nicht notwendig sind. Mit den Verbindungen der Zwischenstufe IV ist eine Vielzahl neuer für die Katalyse interessanter Liganden in einfacher Weise zugänglich geworden. Für die Herstellung einer Ligandenbibliothek der erfindungsgemäßen Liganden ist das erfindungsgemäße Verfahren deutlich konvergenter als bisher bekannte Verfahren.

Die erfindungsgemäß hergestellten Diphosphine können als Liganden in metallkatalysierten Reaktionen, insbesondere Hydroformylierungen von Olefinen mit Rhodium verwendet werden. Bevorzugte Olefine sind Octene und Butene, insbesondere solche mit hohen Verzweigungsgraden und/oder innenliegenden Doppelbindungen.

Die Verwendung von Diphosphinen als Liganden in Hydroformylierungsreaktionen ist bekannt. So werden in US 4 694 109 Diphosphin-Liganden mit BISBI-Grundstruktur genannt, die u.a. auch in der Hydroformylierung von Olefinen mit innenständigen Doppelbindungen eingesetzt werden, ohne dass auf die Selektivität des Produktes und der Aktivität des Katalysators eingegangen wird.

L. A. van der Veen, P. C. J. Kamer, P. W. N. M. van Leeuwen berichten in *Angew. Chem. Int. Ed.* **1999,** 38, 336-338 von modifizierten XANTPHOS Liganden, die 2-Octen zum n-Nonanal mit einem n/i-Verhältnis von 9:1 und einer Turnover-Frequenz (=TOF) von 112 h⁻¹. TOF's ist definiert als das Verhältnis von mol Aldehyd zu mol Rhodium pro h nach einem 20-30% Umsatzes.

EP 0 653 432 beschreibt die Darstellung von NAPHOS-analogen Diphosphinen, die teilweise fluorhaltige Substituenten enthalten. Diese Liganden können in der Hydrofonnylierung von Olefinen eingesetzt werden. Auch hier werden keine Angaben über Selektivität und Aktivität des Katalysators gemacht.

Die erfindungsgemäß hergestellten Liganden - insbesondere die Fluor-haltigen NAPHOS-analogen Liganden - in Metallkomplexe stellen überraschenderweise hochselektive und aktive Katalysatoren dar, die Olefine mit innenständigen Doppelbindungen in der Hydrofonnylierung selektiv zum terminalen Aldehyd mit den bisher bekannten höchsten Aktivitäten umsetzen. Tabelle 1 in den Beispielen zeigt eine Übersicht.

**Die Verwendung von Diphosphinen der Formel** 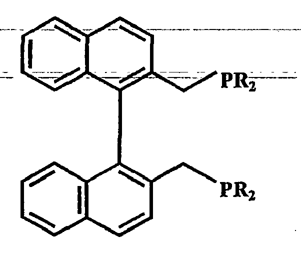 mit
R = 3,5-C₆H₃F₂; -C₆F₅; 2,4,5-C₆H₂F₃, 3,5-C₆H₃(CF₃)₂; 3,5-C₆H₃(CH₃)₂ oder 3,4,5-C₆H₂F₃
als Rhodium-Liganden in einem Verfahren zur Hydroformylierung von Olefinen mit 3 bis 16 C-Atomen unter Rhodium-Katalyse ist ebenfalls Gegenstand der Erfindung, wobei die n/i-Selektivität der erhaltenen Aldehyde über 85 : 15, bevorzugt über 97 : 3, liegt.

Darüber hinaus können in der erfindungsgemäßen Verwendung mit den genannten Liganden Turn-over-frequenzen (TOF), d. h. Aktivitäten von mehr als 150, bevorzugt mehr als 300 erreicht werden.

Die Hydroformylierung kann in üblicher Weise, wie z. B. in J. Falbe et al "New Syntheses with Carbon Monoxide", Springer Verlag 1980, Seite 93-123 beschrieben, durchgeführt werden.
Bevorzugt werden als Olefine solche mit innenständigen Doppelbindungen, wie Buten-2, Raffinat I oder Raffinat II, Penten und/oder Octen, jeweils isomerenrein oder als Isomerengemisch eingesetzt.

Raffinat I und II bezeichnen Crack-C₄-Gemische, die Butane, Butene und Butadiene im Gemisch enthalten.

### Beispiele:

Die nachstehenden Beispiele dienen zur Erläuterung der erfindungsmäßigen Verfahren, ohne die Verfahren auf die Ausübung der Beispiele zu beschränken.

### 1. Allgemeine Arbeitsvorschrift zur Herstellung der Diphosphinen:

Lösungsmittel: Alle verwendeten Lösungsmittel werden nach bekannten Verfahren getrocknet und unter Argon destilliert und aufbewahrt.

### a) Darstellung von 2,2'-Bis-{{[bis-(diethylamino)]phosphinyl}-methyl}-1,1'-binaphthyl

Von 33.2 ml n-BuLi-Lsg. (1.6 M in Hexan, 53 mmol) werden im Vakuum unter leichtem Erwärmen der Großteil des Lösungsmittels abgezogen. Nach Kühlen im Eisbad werden 25 ml Ether sowie 7.9 ml Tetramethylethylendiamin (53 mmol) zugegeben. Eine Lösung von 5 g 2,2'-Dimethyl-1,1'-binaphthyl (17.7 mmol) in 30 ml Ether wird langsam unter Rühren und Kühlen zugetropft. Das Reaktionsgemisch wird 24 h bei Raumtemperatur und anschließend einige Stunden bei 0°C stehen gelassen. Die überstehende Lösung wird abdekantiert und der ausgefallene Niederschlag zweimal mit je 25 ml Hexan gewaschen.

Der tiefrot gefärbte Niederschlag wird mit 50 ml n-Hexan versetzt und auf-70 °C gekühlt. Zu der gerührten Suspension wird eine Mischung von 7.5 ml Chlor-bis-(diethylamino)-phosphin (35.4 mmol) und 25 ml absolutem n-Hexan getropft. Nach langsamer Erwärmung auf Raumtemperatur wird das Gemisch weitere 12 h gerührt. Die Lösung wird filtriert und der Niederschlag zweimal mit je 50 ml Toluol nachextrahiert. Die vereinigten Filtrate werden im Vakuum (10⁻³ Torr) unter Erwärmen auf 80 °C von Lösungsmitteln sowie von überschüssigem Chlor-bis(diethylamino)phosphin befreit. Der Rückstand enthält das Zielprodukt in guter Reinheit und kann ohne weitere Aufreinigung für die weiteren Umsetzungen z.B. zum 2,2'-Bis-[(dichlorphosphinyl)-methyl]-1,1'-binaphthyl verwendet werden.
M=514.59g/mol
³¹P-NMR (δ[ppm], C₆D₆): 88.4
¹H-NMR (δ[ppm], J[Hz], C₆D₆): 0.45 t (= Triplett), J =7 (6H); 0.55 t, J = 7 (6H); 2.5 q (= Quartett), J = 7 (4H); 2.6 q, J = 7 (4H); 2.85 d (= Dublett), J = 3 (4H); 6.7-7.0 m (= Multiplett) (6H); 7.6 d, J = 8 (2H); 7.7 -7.8 m (4H).

### b) Darstellung von 2,2'-Bis-[(dichlorphosphinyl)-methyl]-1,1'-binaphthyl

Das 2,2'-Bis-{{[bis-(diethylamino)]phosphinyl}-metyl}-1,1'-binaphthyl, welches sich im Rückstand der unter 1a) beschriebenen Reaktion (s.o.) befindet, wird in 150 ml Hexan aufgenommen. Unter Rühren und Kühlen im Eisbad wird gasförmiges HCl bis zur Sättigung (ca. 1 h) eingeleitet. Das Gemisch wird filtriert und der Rückstand zweimal mit 25 ml Hexan gewaschen. Die vereinigten Filtrate werden auf etwa 50 ml eingeengt. Bei -30 °C kristallisieren 2.9 g 2,2'-Bis[(dichlorphosphinyl)-methyl]-1,1'-binaphthyl aus. M = 484.12 g/mol
Ausbeute: 2.9 g (44 % Ausbeute bezogen auf das unter la) eingesetzte 2,2'-Dimethyl-1,1 binaphthyl)
³¹P-NMR (δ[ppm], J[Hz], CDCl₃): 180.00
¹H-NMR (δ[ppm], J[Hz], CDCl₃): 3.4 -3.6 m (4H); 7.0 d, J = 8.5 (2H); 7.2 m (2H); 7.43 m (2H); 7.6 d, J = 8.5 (2H); 7.86 d, J = 8.3 (2H); 7.94 d, J = 8.5 (2H) ¹³C-NMR: (δ[ppm], J[Hz], CDCl₃): 50.3 d, ¹J_{PC} = 45.8 (P-CH₂); 126.4 d, J = 5.7; 126.5 d, J = 6.7; 126.9 s; 128.1 s; 128.4 d, J = 2.9; 129.0 s; 129.6 d J = 5; 133.0 d, J = 16.2; 135.0 d, J = 2; 135.6s.

### c) Allgemeine Arbeitsvorschrift zur Synthese der Liganden der Formel (I):

Es werden 302 mg (12.4 mmol) elementares Magnesium unter Argon in einem 100 ml Dreihalskolben versehen mit Magnetrührung mit 10 ml absolutem Diethylether gemischt. Zu diesem Gemisch werden bei Raumtemperatur mittels eines Tropftrichters 12.4 mmol des entsprechenden substituierten Bromverbindung (gelöst in 10 ml Diethylether) langsam (ca. innerhalb einer Stunde) zugetropft. Sollte die Grignardreaktion nicht starten, wird der Reaktionslösung ein Tropfen 1,2-Dibromethan zugesetzt. Wenn die Grignardreaktion (vollständiger Umsatz des Magnesiums) nach ca. 1 Stunde beendet ist, wird diese Lösung in einen Tropftrichter überführt.
1.5 g (3.1 mmol) des 2,2'-Bis-[(dichlorphosphinyl)-methyl]-1,1'-binaphthyls werden in einem 150 ml Dreihalskolben unter Argon in 25 ml absolutem THF gelöst.
Die Grignardlösung wird bei Raumtemperatur langsam (innerhalb einer halben Stunde) zu der Lösung des 2,2'-Bis-[(dichlorphosphinyl)-methyl]- 1,1'-binaphthyls zugetropft.
Anschließend wird die Lösung zum Sieden erhitzt und noch zwei Stunden zur Vervollständigung der Reaktion gerührt. Unter Argon wird anschließend am Rotationsverdampfer bis zur Trockene eingeengt. Der Rückstand wird in 50 ml absolutem und/oder entgastem Toluol gelöst und mit 20 ml entgastem Wasser versetzt. Es wird eine halbe Stunde gerührt. Die wäßrige Phase wird abgetrennt und die organische Phase wird mit Natriumsulfat getrocknet.

Anschließend wird das Natriumsulfat abfiltriert, mit 25 ml Toluol gewaschen, die organische Phase im Vakuum ins Trockene eingeengt. Der Rückstand wird aus Aceton/EtOH, Aceton/MeOH oder Toluol/Hexan umkrisallisiert, oder unter Argon über eine 40 cm Säule (Kieselgur G 60) mit absolutem Toluol als Eluenten säulenchromatografisch gereinigt.

Folgende Liganden wurden nach dem erfindungsgemäßen Verfahren hergestellt.

### 2.) Beispiele der gemäß 1c) dargestellten Liganden:

### a) 2,2'-Bis-{{bis-[(2,3,4,5,6-pentafluor-phenyl)]-phosphinyl}-methyl}-1,1'-binaphthyl

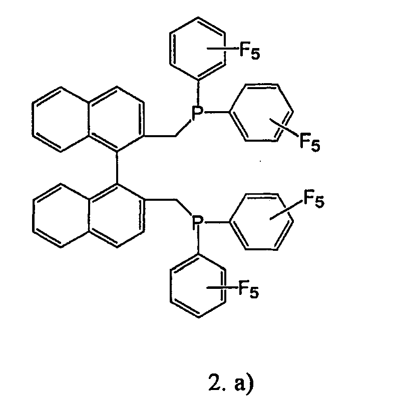

M = 1010.54 g/mol
Ausbeute: 2.3 g (73.4 % d. Theorie)
³¹P-NMR (δ[ppm], J[Hz], CDCl₃): 46.3 qui (Quintett), 3^{J}_{PF}- 11 ¹H-NMR: (δ[ppm], J[Hz], CDCl₃): 3.6 d, J = 13.8 (2H); 4.1 d, J = 13.8 (2H); 6.3 d, J = 8.4 (2H); 6.85 t, J = 17; 7.25 t, J =10.8 (2H); 7.65 d, J = 8.6 (2H); 7.7 d, J = 8.1 (2H); 7.94 d, J = 8.6 (2H).

### b) 2,2'-Bis-{{bis-[(3,5-difluor-phenyl)]-phosphinyl}-metlryl}-1,1'-binaphthyl

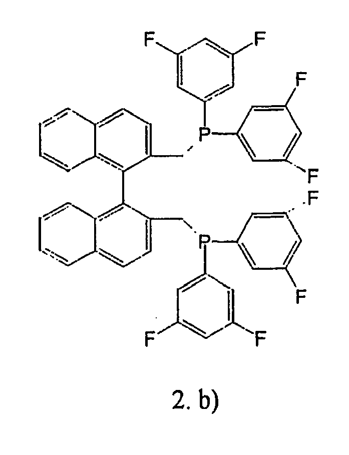

M = 794.66 g/mol
Ausbeute: 2.05 g (83.2 % d. Theorie)
³¹P-NMR (δ[ppm], J[Hz], CDCl₃): -8.99
¹H-NMR (δ[ppm], J[Hz], CDCl₃): 3.05- 3.1 m, (4H); 6.3 tt (= Triplett vom Triplett), J = 6, J = 2.2 (4H); 6.53 tt, J = 8.9, J = 2.2 (2H); 6.65 m, (6H); 6.88 d, J = 8.5 (2H); 7.1 tt, J = 7 ,J = 1 (2H); 7.2 d, J = 8.5 (2H); 7.31 t, J = 7.1 (2H); 7.78 t, J = 8.5 (4H).

### c) 2,2'-Bis-{{bis-[(2,4,5-trifluor-phenyl)]-phosphinyl}-methyl}-1,1'-binaphthyl

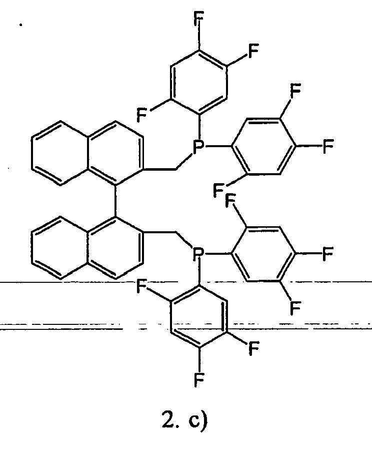

M = 866.62 g/mol
Ausbeute: 1.3 g (48.4 % d. Theorie)
³¹P-NMR (δ[ppm], J[Hz], CDCl₃): -28.8 d, J = 23.6; -29.1 d, J = 25.0
¹H-NMR (δ[ppm], J[Hz], CDCl₃): 3.26 d, J_{HH} = 14.1 (2H); 3.45 dd, J_{HH} = 14, ²J_{PH}= 3.2 (2H); 6.33 m (2H); 6.55 d; 8.7 m (2H); 6.6-6.8 m (6 H); 6.92 td (= Triplett vom Dublett), J = 6.9 J = 1.2 (2H); 7.26 t, J = 7.4 (2H); 7.43 dd, J = 8.5, J = 2.2 ( 2 H); 7.7 d, J = 8.1 (2H); 7.76 d, J = 7.7 (2H).

### d) 2,2'-Bis-{{bis-[(3,5-dimethyl-phenyl)]-phosphinyl}-methyl}-1,1'-binaphthyl

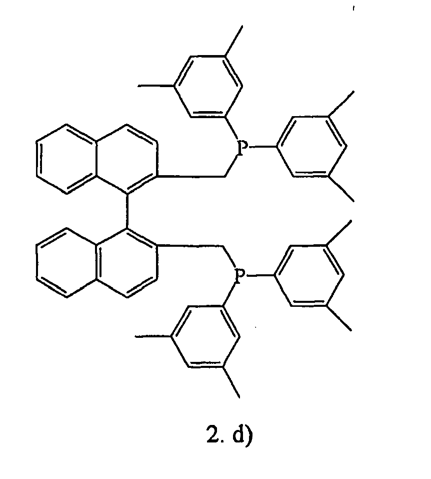

M = 762.95 g/mol
Ausbeute: 1.9 g ( 80% d. Theorie)
³¹P-NMR (δ[ppm], J[Hz], CDCl₃): -11.4
¹H-NMR (δ[ppm], J[Hz], CDCl₃): 2.1 s, 2,2 s, 2.3 s (24H); 3.1 dd, J_{HH}= 14.2, J = 2.5 (2H); 3.3 dd, J_{HH} = 14.2, ²J_{PH} = 2.2 (2H); 6.6 d, J = 7.5 (4H); 6.9 m (6H), 7.1-7.2 m (6H); 7.35 dd, J = 8.5, J = 2 (2 H); 7.4 t, J = 7 (2H); 7.8 d, J = 8.5 (2H); 7.85 d, J = 7.9 (2H).

### e) 2,2'-Bis-{{bis-[(3,4,5-trifluor-phenyl)]-phosphinyl}-methyl}-1,1'-binaphthyl

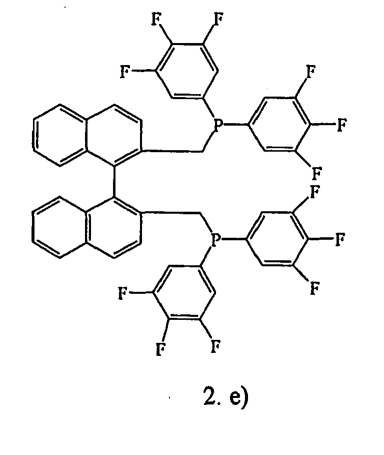

M = 866.62 g/mol
Ausbeute: 2.3 g (85% d. Theorie)
³¹P-NMR (δ[ppm], J[Hz], CDCl₃): -8.5
¹H-NMR (δ[ppm], J[Hz], CDCl₃): 3.1 m, (2H), 3.05 dd, J_{HH} = 13.8, ²J_{PH} = 2.7 (2H); 6.3 q, J = 6.7 (4H); 6.7 m (6H); 7.05 td, J = 6.7, J = 1.3 (2H); 7.3 m(4H), 7.8 t, J = 8.9 (4H).

### f) 2,2'-Bis-{{bis-[(3,5-trifluormethyl-phenyl)]-phosphinyl}-methyl}}-1,1'-binaphthyl

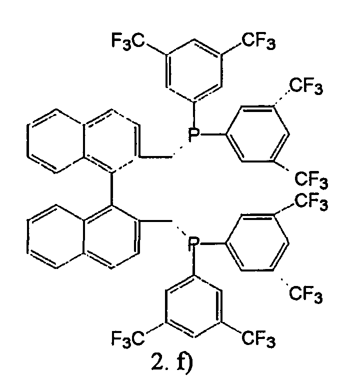

M = 1126.72 g/mol
Ausbeute: 3.1 g (88.7 % d. Theorie)
³¹P-NMR (δ[ppm], J[Hz], CDCl₃): -10.5
¹H-NMR (δ[ppm], J[Hz], CDCl₃): 3.2 m, (4H); 6.95 d, J = 8.5 (2H); 7.05-7.15 m, (4H); 7.25 d, J = 4.7 (4H); 7.33 t, J = 7.3 (2H); 7.47 d, J = 4.5 (4H); 7.65 s, (2H); 7.7 d, J = 11.1 (2H); 7.8 d, J = 16.5 (4H).

### 3. Beschreibung der Hydroformylierung

Die Hydroformylierungsexperimente wurden entweder in einem 100 ml Autoklaven (Parr Co.) mit einem Magnet-betriebenen Propeller-Rührer oder in einem 160 ml Autoklaven (Parr Co.) mit einem Magnetrührer durchgeführt.

Anhand des Autoklavenversuches mit der Nr. 1 (siehe Tabelle 1) wird die Durchführung der Hydroformylierung beispielshaft beschrieben:

Zu einer Lösung aus 1.88 mg Rh(CO)₂acac (7,3 µmol) und 44 mg vom Ligand **2.f**) (M: 1127, 36,5 µmol) in Anisol (30 ml) wird Isooctan (2 ml als innerer Standard) and 1-Penten (8 ml, 73.0 mmol) gegeben. Nachdem-die Mischung in den Autoklaven überführt wurde, wird der Autoklav mit Synthesegas (CO : H₂ = 1 : 1) auf 5 bar gebracht. Daraufhin wird zur langsamen Druckanpassung zunächst auf 120 °C erhitzt und anschließend der Druck auf 10 bar nachreguliert. Nach 16 h wird der Autoklav automatisch abgeschalten, mit einem Eisbad gekühlt und auf Normaldruck entspannt. Eine GC-Probe wurde direkt aus dem Autoklaven entnommen und vermessen.

**Tabelle 1: Rhodium-katalysierte Hydroformylierung in Gegenwart der erfindungsgemäßen Liganden**

| Nr. | Ligand | Olefin | T (°C) | Ausbeute (%) | n/i | TOF |
|---|---|---|---|---|---|---|
| 1 | 2f | 1-Penten | 120 | 82 | 96:4 | 512 |
| 2 | 2f | 2-Penten | 120 | 68 | 91:9 | 425 |
| 3 | 2f | 2-Penten | 100 | 52 | 89:11 | 325 |
| 4 | 2f | 2-Buten | 120 | 66 | 91:9 | 825 |
| 5 | 2f | 2-Octen | 120 | 51 | 86:14 | 319 |
| 6 | 2b | 1-Penten | 120 | 78 | 97:3 | 488 |
| 7 | 2b | 2-Penten | 120 | 59 | 94: 6 | 369 |
| 8 | 2b | 2-Penten | 100 | 21 | 95:5 | 131 |
| 9 | 2e | 1-Penten | 120 | 83 | 97:3 | 519 |
| 10 | 2e | 2-Penten | 120 | 61 | 93:7 | 381 |
| 11 | 2e | 2-Penten | 100 | 24 | 94:6 | 150 |
| 12 | 2e | 2-Buten | 120 | 74 | 95:5 | 925 |
| 13 | 2e | 2-Octene | 120 | 48 | 91:9 | 300 |
| 14 | 2e | 4-Octen | 120 | 14 | 66:34 | 88 |
| 15 | 2d | 1-Penten | 120 | 76 | 81:19 | 475 |
| 16 | 2d | 2-Penten | 120 | 11 | 78:22 | 69 |

Ein Katalysator, der mit NAPHOS - dem Grundkörper ohne Fluor-haltige Substituenten - gebildet wird, zeigt - wie in der folgenden Tabelle ersichtlich - zwar ebenfalls hohe Selektivitäten, allerdings geringe Aktivitäten, welche anhand der TOF-Zahlen ablesbar sind.

**Tabelle 2 : Hydroformylierung von 1- und 2-Penten mit NAPHOS**

| **Nr.** | **Olefin** | **P (bar)** | **T (°C)** | **Ausbeute (%)** | **n/i** | **TOF** |
|---|---|---|---|---|---|---|
| 1 | 1-Penten | 10 | 120 | 76 | 99:1 | 475 |
| 2 | 1-Penten | 50 | 120 | 88 | 97:3 | 550 |
| 3 | 2-Pcnten | 10 | 120 | 22 | 89:11 | 138 |
| 4 | 2-Penten | 50 | 120 | 7 | 55:45 | 44 |

## Patentansprüche

1. Verfahren zur Herstellung von Diphosphinen der allgemeinen Formel I
R¹R²P-H₂C-Ar-CH₂-PR¹R² (I)
**dadurch gekennzeichnet,**
**dass**
a) Dimethylverbindungen der allgemeinen Formel II,
H₃C-Ar-CH₃ (II)
mit einer Base und einem N-substituierten Aminophosphorhalogenid zur Bis-(aminophosphinyl-methyl)-verbindung der allgemeinen Formel III
R³R⁴P-H₂C-Ar-CH₂PR³R⁴, (III)
b) die so erhaltenen Verbindungen der allgemeinen Formel III mit HCl zur Bis(dichlorphosphinyl-methyl)-Verbindung der allgemeinen Formel IV
Cl₂P-H₂C-Ar-CH₂-PCl₂ (IV)
und c) die so erhaltenen Verbindungen der allgemeinen Formel IV mit metallorganischen Reagenzien zu den Zielverbindungen der allgemeinen Formel I umgesetzt werden, wobei R¹ und R² unabhängig voneinander für einen substituierten oder unsubstituierten aromatischen, heteroaromatischen oder aliphatischen Kohlenwasserstoffrest stehen, die eine kovalente Bindung zueinander aufweisen können und Ar für einen substituierten oder unsubstituierten aromatischen oder heteroaromatischen Kohlenwasserstoffrest und R³ und R⁴ unabhängig voneinander für einen N-substituierten Aminoalkyl- oder Aminoarylrest stehen.

2. Verfahren nach Anspruch 1,
**dadurch gekennzeichnet,**
**dass** die Reste R¹ und R² jeweils gleich oder verschieden sind und für Aromaten mit bis zu 14 C-Atomen stehen, die bis zu acht Substituenten aus der Gruppe (C₁-C₈)-Alkyl, O-Alkyl-(C₁-C₈), O-Phenyl, Phenyl, Fluor, Chlor, CN, COOH, CHO, SO₃H, SO₂-Alkyl-(C₁₋C₆), SO-Alkyl-(C₁-C₆), CF₃, COO-Alkyl-(C₁-C₈), COO-Phenyl, PO-Phenyl₂, POAlkyl₂₋(C₁-C₄), PO₃H₂, PO(O-Alkyl-(C₁-C₆))₂, SO₃-Alkyl-(C₁-C₄) tragen können.

3. Verfahren nach einem der Ansprüche 1 oder 2,
**dadurch gekennzeichnet,**
**dass** die Reste R¹ und R² jeweils gleich oder verschieden sind und für Heteroaromaten mit einem fünf-, sechs- oder siebengliedrigen Ring mit Stickstoff-, Sauerstoff- oder Schwefelatomen im Ring, wobei an den Ring weitere aromatische, heteroaromatische und/oder aliphatische Ringe ankondensiert sein und die Ringe bis zu sieben Substituenten aus der Gruppe (C₁-C₈)-Alkyl, O-Alkyl-(C₁-C₈), O-Phenyl, Phenyl, Fluor, Chlor, CN, COOH, CHO, SO₃H, SO₂-Alkyl-(C₁-C₆), SO-Alkyl-(C₁-C₆), CF₃, COO-Alkyl-(C₁-C₈), COO-Phenyl, PO-Phenyl₂, POAlkyl₂-(C₁-C₄), PO₃H₂, PO(O-Alkyl-(C₁-C₆))₂, SO₃-Alkyl-(C₁-C₄) tragen können, stehen.

4. Verfahren nach einem der Ansprüche 1 bis 3,
**dadurch gekennzeichnet,**
**dass** die Kohlenwasserstoffreste R¹ und R² jeweils gleich oder verschieden sind und für bis zu 10 C-Atomen enthaltende cyclische oder acyclische Alkylreste stehen, die bis zu sieben Substituenten aus der Gruppe (C₁-C₈)-Alkyl, O-Alkyl-(C₁-C₈), O-Phenyl, Phenyl, Fluor, Chlor, CN, COOH, CHO, SO₃H, SO₂-Alkyl-(C₁-C₆), SO-Alkyl-(C₁-C₆), CF₃, COO-Alkyl-(C₁-C₈), COO-Phenyl, PO-Phenyl₂, POAlkyl₂-(C₁-C₄), PO₃H₂, PO(O-Alkyl-(C₁-C₆))₂, SO₃-Alkyl-(C₁-C₄) tragen können.

5. Verfahren nach einem der Ansprüche 1 bis 4,
**dadurch gekennzeichnet,**
**dass** die Reste R¹ und R² eine oder mehrere kovalente Verbindungen zueinander aufweisen.

6. Verfahren nach einem der Ansprüche 1 bis 5,
**dadurch gekennzeichnet,**
**dass** Ar für einen Phenyl-, Naphthyl-, Anthracenyl- oder Phenanthrenylrest, eine 1,1'-Biphenyleinheit oder eine 1,1'-Binaphthyleinheit steht, die jeweils bis zu acht weitere Substituenten aus der Gruppe (C₁-C₈)-Alkyl, O-Alkyl-(C₁-C₈), O-Phenyl, Phenyl, Fluor, Chlor, CN, COOH, CHO, SO₃H, SO₂-Alkyl-(C₁-C₆), SO-Alkyl-(C₁-C₆), CF₃, COO-Alkyl-(C₁-C₈), COO-Phenyl, PO-Phenyl₂, POAlkyl₂-(C₁-C₄), PO₃H₂, PO(O-Alkyl-(C₁-C₆))₂, SO₃-Alkyl-(C₁-C₄) oder einen oder mehrere ankondensierte Phenylringe tragen können.

7. Verfahren nach einem der Ansprüche 1 oder 6,
**dadurch gekennzeichnet,**
**dass** Ar für Heteroaromaten mit einem fünf-, sechs- oder siebengliedrigen Ring mit Stickstoff-, Sauerstoff- oder Schwefelatomen im Ring, wobei an den Ring weitere aromatische, heteroaromatische und/oder aliphatische Ringe ankondensiert sein und die Ringe bis zu sechs Substituenten aus der Gruppe (C₁-C₈)-Alkyl, O-Alkyl-(C₁-C₈), O-Phenyl, Phenyl, Fluor, Chlor, CN, COOH, CHO, SO₃H, SO₂-Alkyl-(C₁-C₆), SO-Alkyl-(C₁-C₆), CF₃, COO-Alkyl-(C₁-C₈), COO-Phenyl, PO-Phenyl₂, POAlkyl₂-(C₁-C₄), PO₃H₂, PO(O-Alkyl-(C₁-C₆))₂, SO₃-Alkyl-(C₁-C₄) tragen können, steht.

8. Verfahren nach einem der Ansprüche 1 bis 7,
**dadurch gekennzeichnet,**
**dass** als Basen in Verfahrensschritt a)
Alkyl- oder Aryllithium-Verbindungen, Alkali- oder Erdalkalihydride, Alkali- oder Erdalkalimetalle in flüssigem Ammoniak, Alkali- oder Erdalkalihydroxide oder Alkali- oder Erdalkalialkoxide eingesetzt werden.

9. Verfahren nach einem der Ansprüche 1 bis 8,
**dadurch gekennzeichnet**,
als N-substituierte Aminophosphorhalogenide im Verfahrensschritt a) Alkyl- oder Arylaminophosphorchloride, -bromide oder -iodide eingesetzt werden, wobei für Alkyl ein Kohlenwasserstoffrest aus der Gruppe Methyl-, Ethyl-, Propyl-, Isopropyl-, Butyl-, Isobutyl-, Hexyl-, 2-Ethylhexyl-, Octyl-, Isooctyl-, Nonyl- oder Isononyl-Rest und/oder diese Reste zueinander kovalente Verbindungen aufweisen und/oder für Alkylamino ein bis zu 6 C-Atomen enthaltender aliphatischer heterocyclischer Rest und/oder für Aryl ein bis zu 14 C-Atomen enthaltender aromatischer Rest stehen können, wobei diese Reste bis zu sieben Substituenten aus der Gruppe (C₁-C₈)-Alkyl, O-Alkyl-(C₁-C₈), O-Phenyl, Phenyl, Fluor, Chlor, CN, COOH, CHO, SO₃H, SO₂-Alkyl-(C₁-C₆), SO-Alkyl-(C₁-C₆), CF₃, COO-Alkyl-(C₁-C₈), COO-Phenyl, PO-Phenyl₂, POAlkyl₂-(C₁-C₄), PO₃H₂, PO(O-Alkyl-(C₁-C₆))₂, SO₃-Alkyl-(C₁-C₄) tragen können.

10. Verfahren nach einem der Ansprüche 1 bis 9,
**dadurch gekennzeichnet,**
**dass** als metallorganische Reagenzien im Verfahrensschritt c) Übergangsmetallarene, aliphatische oder aromatische Alkali- oder Erdalkaliverbindungen, aliphatische oder aromatische Grignardreagenzien, Alkyl- oder Arylzinkreagenzien, Alkyl- oder Aryllithiumreagenzien und/oder Arylkupferverbindungen eingesetzt werden.

11. Verwendung von Diphosphinen der Formel 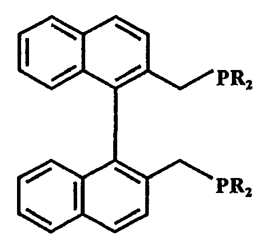 mit
R = 3,5 - C₆H₃F₂; -C₆F₅; 2,4,5 -C₆H₂F₃, 3,5-C₆H₃(CF₃)₂; 3,5 - C₆H₃(CH₃)₂ oder 3,4,5-C₆H₂F₃
als Rhodium-Liganden in einem Verfahren zur Hydroformylierung von Olefinen mit 3 bis 16 C-Atomen unter Rhodium-Katalyse, wobei die n/i-Selektivität der erhaltenen Aldehyde über 85 : 15 liegt.

12. Verwendung nach Anspruch 11,
**dadurch gekennzeichnet,**
**dass** als Olefin, Buten, Raffinat I, Raffinat II, Penten und/oder Octen, jeweils isomerenrein oder als Isomerengemisch verwendet werden.

## Claims

1. A process for preparing a diphosphine of the general formula I
R¹R²P-H₂C-Ar-CH₂PR¹R² (I)
**characterized in that**
a) a dimethyl compound of the general formula II,
H₃C-Ar-CH₃ (II)
is reacted with a base and an N-substituted aminophosphorus halide to form a bis(aminophosphinomethyl) compound of the general formula III
R³R⁴P-H₂C-Ar-CH₂-PR³R⁴, (III)
b) the compound of the general formula III obtained in this way is reacted with HCl to form a bis(dichlorophosphinomethyl) compound of the general formula IV
Cl₂P-H₂C-Ar-CH₂-PCl₂ (IV)
and c) the compound of the general formula IV obtained in this way is reacted with an organometallic reagent to give the target compound of the general formula I, where R¹ and R² are each, independently of one another, a substituted or unsubstituted aromatic, heteroaromatic or aliphatic hydrocarbon radical and may have a covalent bond connecting them and Ar is a substituted or unsubstituted aromatic; or heteroaromatic hydrocarbon radical and R³ and R⁴ are each, independently of one another, an N-substituted alkylamino or arylamino radical.

2. A process according to claim 1, **characterized in that** the radicals R¹ and R² are identical or different and are each an aromatic having up to 14 carbon atoms and may bear up to eight substituents selected from the group consisting of (C₁-C₈)-alkyl, O-alkyl-(C₁-C₈), O-phenyl, phenyl, fluorine, chlorine, CN, COOH, CHO, SO₃H, SO₂-alkyl-(C₁-C₆), SO-alkyl-(C₁-C₆), CF₃, COO-alkyl-(C₁-C₈), COO-phenyl, PO-phenyl₂, PO-alkyl₂- (C₁-C₄), PO₃H₂, PO- (O-alkyl- (C₁-C₆))₂, SO₃-alkyl-(C₁-C₄),

3. A process according to either one of claims 1 and 2, **characterized in that** the radicals R¹ and R² are identical or different and are each a heteroaromatic having a five-, six- or seven-membered ring and nitrogen, oxygen or sulfur atoms in the ring, where further aromatic, heteroaromatic and/or aliphatic rings may be fused onto the ring and the rings may bear up to seven substituents selected from the group consisting of (C₁-C₈)-alkyl, O-alkyl-(C₁-C₈), O-phenyl, phenyl, fluorine, chlorine, CN, COOH, CHO, SO₃H, SO₃alkyl-(C₁-C₆), SO-alkyl-(C₁-C₆), CF₃, COO-alkyl- (C₁-C₈), COO-phenyl, PO-phenyl₂, PO-alkyl₂- (C₁-C₄), PO₃H₂, PO-(O-alkyl-(C₁-C₆))₂, SO₃-alkyl- (C₁-C₄).

4. A process according to any one of claims 1 to 3, **characterized in that** the hydrocarbon radicals R¹ and R² are identical or different and are each a cyclic or acyclic alkyl radical which contains up to 10 carbon atoms and may bear up to seven substituents selected from the group consisting of (C₁-C₈)-alkyl, O-alkyl-(C₁-C₈), 0-phenyl, phenyl, fluorine, chlorine, CN, COOH, CHO, SO₃H, SO₂-alkyl-(C₁-C₆), SO-alkyl-(C₁-C₆), CF₃, COO-alkyl-(C₁-C₈), COO-phenyl, PO-phenyl₂, PO-alkyl₂- (C₁-C₄) PO₃H₂, PO-(O-alkyl- (C₁-C₆))₂, SO₃-alkyl-(C₁-C₄).

5. A process according to any one of claims 1 to 4, **characterized in that** the radicals R¹ and R² have one or more covalent bonds between them.

6. A process according to any one of claims 1 to 5, **characterized in that** Ar is a phenyl, naphthyl, anthracenyl or phenanthrenyl radical, a 1,1'-biphenyl unit or a 1,1'-binaphthyl unit, which may each bear up to eight further substituents selected from the group consisting of (C₁-C₈)-alkyl, O-alkyl-(C₁-C₈), O-phenyl, phenyl, fluorine, chlorine, CN, COOH, CHO, SO₃H, SO₂-alkyl- (C₁-C₆), SO-alkyl-(C₁-C₆), CF₃, COO-alkyl-(C₁-C₈), COO-phenyl, PO-phenyl₂, PO-alkyl₂-(C₁-C₄), PO₃H₂, PO-(O-alkyl-(C₁-C₆))₂, SO₃-alkyl-(C₁-C₄) or one or more fused-on benzene rings.

7. A process according to any one of claims 1 to 5, **characterized in that** Ar is a heteroaromatic comprising a five-, six- or seven-membered ring having nitrogen, oxygen or sulfur atoms in the ring, where further aromatic, heteroaromatic and/or aliphatic rings may be fused onto the ring and the rings may bear up to six substituents selected from the group consisting of (C₁-C₈) - alkyl, O-alkyl-(C₁-C₈), 0-phenyl, phenyl, fluorine, chlorine, CN, COOH, CHO, SO₃H, SO₂-alkyl-(C₁-C₆), SO-alkyl- (C₁-C₆), CF₃, COO-alkyl- (C₁-C₈), COO-phenyl, PO-phenyl₂, PO-alkyl₂-(C₁-C₄), PO₃H₂, PO- (O-alkyl- (C₁-C₆))₂, SO₃-alkyl- (C₁-C₄).

8. A process according to any one of claims 1 to 7, **characterized in that** an alkylithium or aryllithium compound, an alkali metal hydride or alkaline earth metal hydride, an alkali metal or alkaline earth metal in liquid ammonia, an alkali metal hydroxide or alkaline earth metal hydroxide or an alkali metal alkoxide or alkaline earth metal alkoxide is used as base in process step a).

9. A process according to any one of claims 1 to 8, **characterized in that** an alkylaminophosphorus or arylaminophosphorus chloride, bromide or iodide, where alkyl is a hydrocarbon radical selected from the group consisting of methyl, ethyl, propyl, isopropyl, butyl, isobutyl, hexyl, 2-ethylhexyl, octyl, isooctyl, nonyl or isononyl radicals and/or these radicals can have a covalent bond between them and/or alkylamino is an aliphatic heterocyclic radical having from one to 6 carbon atoms and/or aryl is an aromatic radical having up to 14 carbon atoms, where these radicals may bear up to seven substituents selected from the group consisting of (C₁-C₈)-alkyl, O-alkyl-(C₁-C₈), O-phenyl, phenyl, fluorine, chlorine, CN, COOH, CHO, SO₃H, SO₂-alkyl- (C₁-C₆), SO-alkyl-(C₁-C₆), CF₃, COO-alkyl- (C₁-C₈), COO-phenyl, PO-phenyl₂, PO-alkyl₂ (C₁-C₄), PO₃H₂, PO (O-alkyl-(C₁-C₆))₂, SO₃-alkyl- (C₁-C₄), is used as N-substituted aminophosphorus halide in process step a).

10. A process according to any one of claims 1 to 9, **characterized in that** a transition metal arene, an aliphatic or aromatic alkali metal compound or alkaline earth metal compound, an aliphatic or aromatic Grighard reagent, an alkylzinc or arylzinc reagent, an alkyllithium or aryllithium reagent and/or an arylcopper compound is used as organometallic reagent in process step c).

11. The use of a diphosphine of the formula 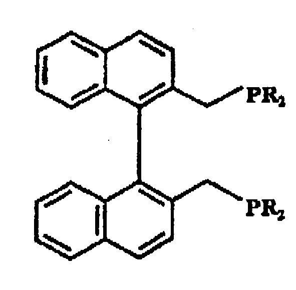 where
R =3, 5-C₆H₃F₂; -C₆F₅; 2, 4, 5-C₆H₂F₃, 3, 5-C₆H₃(CF₃)₂; 3,5-C₆H₃(CH₃)₂ or 3,4,5-C₆H₂F₃,
as rhodium ligand in a process for the hydroformylation of olefins having from 3 to 16 carbon atoms in the presence of a rhodium catalyst, the n/i selectivity of the aldehyde obtained being above 85:15.

12. The use according to claim 11, **characterized in that** butene, raffinate I, raffinate II, pentene and/or octene are/is used as olefin, in each case in isomerically pure form or as a mixture of isomers.

## Revendications

1. Procédé de production de diphosphines de formule générale I,
R¹R²P-H₂C-Ar-CH₂-PR¹R² (I)
**caractérisé en ce que**
a) des composés diméthyliques de formule générale II,
H₃C-Ar-CH₃ (II)
sont mis en réaction avec une base et un halogénure d'amino-phosphore substitué par N pour donner le composé bis-(aminophosphinyl-méthyle) répondant à la formule générale III,
R³R⁴P-H₂C-Ar-CH₂-PR³R⁴ (III)
b) les composés ainsi obtenus, répondant à la formule générale III, sont mis en réaction avec HCl pour donner le composé bis(dichlorophosphinyl-méthyle) de formule générale IV,
Cl₂P-H₂C-Ar-CH₂-PCl₂ (IV)
et
c) les composés ainsi obtenus, répondant à la formule générale IV, sont mis en réaction avec des réactifs organométalliques pour donner les composés visés de formule générale I, dans laquelle
R¹ et R² indépendamment l'un de l'autre représentent un radical d'hydrocarbure aromatique, hétéroaromatique ou aliphatique substitué ou non substitué et peuvent présenter une liaison covalente l'un par rapport à l'autre,
Ar représente un radical d'hydrocarbure radical aromatique ou hétéroaromatique substitué ou non substitué et
R³ et R⁴ indépendamment l'un de l'autre représentent un radical aminoalkyle ou aminoaryle substitué par N.

2. Procédé selon la revendication 1,
**caractérisé en ce que**
les radicaux R¹ et R² sont respectivement identiques ou différents et représentent des composés aromatiques ayant jusqu'à 14 atomes de C, qui peuvent porter jusqu'à huit substituants provenant du groupe alkyle en (C₁-C₈), O-alkyl-(C₁-C₈), O-phényle, phényle, fluore, chlore, CN, COOH, CHO, SO₃H, SO₂-alkyl-(C₁-C₆), SO-alkyl-(C₁-C₆), CF₃, COO-alkyl-(C₁-C₈), COO-phényle, PO-phényl₂, POakyl₂-(C₁-C₄), PO₃H₂, PO(O-alkyl-(C₁-C₆))₂, SO₃-alkyl-(C₁-C₄).

3. Procédé selon l'une des revendications 1 ou 2,
**caractérisé en ce que**
les radicaux R¹ et R² sont respectivement identiques ou différents et représentent des composés hétéroaromatiques comportant un cycle à cinq, six ou sept chaînons, avec des atomes d'azote, d'oxygène ou de soufre dans le cycle, d'autres cycles aromatiques, hétéroaromatiques et/ou aliphatiques pouvant être condensés sur le cycle et les cycles pouvant porter jusqu'à sept substituants provenant du groupe alkyle en (C₁-C₈), O-alkyl-(C₁-C₈), O-phényle, phényle, fluore, chlore, CN, COOH, CHO, SO₃H, SO₂-alkyl-(C₁-C₆), SO-alkyl-(C₁-C₆), CF₃, COO-alkyl-(C₁-C_{S}), COO-phényle, PO-phényl₂, POalkyl₂-(C₁-C₄), PO₃H₂, PO(O-alkyl-(C₁₋C₆))₂, SO₃-alkyl-(C₁-C₄).

4. Procédé selon l'une des revendications 1 à 3,
**caractérisé en ce que**
les radicaux d'hydrocarbure R¹ et R² sont respectivement identiques ou différents et représentent des radicaux alkyle cycliques ou acycliques contenant jusqu'à 10 atomes de C, qui peuvent porter jusqu'à sept substituants provenant du groupe alkyle en (C₁-C₈), O-alkyl-(C₁-C₈), O-phényle, phényle, fluor, chlore, CN, COOH, CHO, SO₃H, SO₂-alkyl-(C₁₋C₆), CO alkyl (C₁ C₀), CF₃, COO alkyl (C₁ C₀), COO-phényle, PO-phényl₂, POalkyl₂-(C₁-C₄), PO₃H₂, PO(O-alkyl-(C₁-C₆))₂, SO₃-alkyl-(C₁₋C₄).

5. Procédé selon l'une des revendications 1 à 4,
**caractérisé en ce que**
les radicaux R¹ et R² présentent une ou plusieurs liaisons covalentes l'un par rapport à l'autre.

6. Procédé selon l'une des revendications 1 à 5,
**caractérisé en ce que**
Ar représente un radical phényle, naphtyle, anthracényle ou phénanthrényle, une unité 1,1'-biphényle ou une unité 1, 1'-binaphtyle qui peuvent porter respectivement jusqu'à huit autres substituants provenant du groupe alkyle en (C₁-C₈), O-alkyl-(C₁₋C₈), O-phényle, phényle, fluor, chlore, CN, COOH, CHO, SO₃H, SO₂-alkyl-(C₁-C₆), SO-alkyl-(C₁-C₆), CF₃, COO-alkyl-(C₁-C₈), COO-phényle, PO-phényl₂, POalkyl₂(C₁-C₄), PO₃H₂, PO(O-alkyl-(C₁₋C₆))₂, SO₃-alkyl-(C₁-C₄) ou un ou plusieurs cycles phényle condensés dessus.

7. Procédé selon l'une des revendications 1 à 5,
**caractérisé en ce que**
Ar représente des composés hétéroaromatiques comportant un cycle à cinq, six ou sept chaînons, avec des atomes d'azote, d'oxygène ou de soufre dans le cycle, d'autres cycles aromatiques, hétéroaromatiques et/ou aliphatiques pouvant être condensés sur le cycle et les cycles pouvant porter jusqu'à sept substituants provenant du groupe alkyle en (C₁-C₈), O-alkyl-(C₁-C₈), O-phényle, phényle, fluor, chlore, CN, COOH, CHO, SO₃H, SO₂-alkyl-(C₁-C₆), SO-alkyl-(C₁-C₆), CF₃, COO-alkyl-(C₁-C₈), COO-phényle, PO-phényl₂, POalkyl₂-(C₁-C₄), PO₃H₂, PO(O-alkyl-(C₁-C₆))₂, SO₃-alkyl-(C₁-C₄).

8. Procédé selon l'une des revendications 1 à 7,
**caractérisé en ce que**
comme base dans l'étape a) du procédé, on utilise des composés de lithium alkylique ou arylique, des hydrures alcalins ou alcalino-terreux, des métaux alcalins ou alcalino-terreux dans de l'ammoniac liquide, des hydroxydes alcalins ou alcalino-terreux ou des alcoxydes alcalins ou alcalino-terreux.

9. Procédé selon l'une des revendications 1 à 8,
**caractérisé en ce que**
comme halogénures d'alkylamino ou d'arylamino-phosphore substitués par N dans l'étape a) du procédé, on utilise des chlorures, bromures ou iodures d'alkyl- ou arylamino-phosphore, dans lesquels alkyle est représenté par un radical d'hydrocarbure du groupe composé de radical méthyle, éthyle, propyle, isopropyle, butyle, isobutyle, hexyle, 2-éthylhexile, octyle, isooctyle, nonyle ou isononyle, et/ou ces radicaux présentent des liaisons covalentes les uns par rapport aux autres, et/ou alkylamino est représenté par un radical hétérocyclique aliphatique contenant jusqu'à 6 atomes de C et/ou aryle par un radical aromatique contenant jusqu'à 14 atomes de C, ces radicaux pouvant porter jusqu'à sept substituants provenant du groupe alkyle en (C₁-C₈), O-alkyl-(C₁₋C₈), O-phényle, phényle, fluor, chlore, CN, COOH, CHO, SO₃H, SO₂₋alkyl-(C₁-C₆), SO-alkyl-(C₁-C₆), CF₃, COO-alkyl-(C₁-C₈), COO-phényle, PO-phényl₂, POalkyl₂-(C₁-C₄), PO₃H₂, PO(O-alkyl-(C₁-C₆))2, SO₃-alkyl-(C₁-C₄).

10. Procédé selon l'une des revendications 1 à 9,
**caractérisé en ce que**
comme réactifs organométalliques dans l'étape c) du procédé, on utilise des arènes de métaux transitoires, des composés alcalins ou alcalino-terreux aliphatiques ou aromatiques, des réactifs de Grignard aliphatiques ou aromatiques, des réactifs alkyliques ou aryliques à base de zinc, des réactifs alkyliques ou aryliques à base de lithium et/ou des composés aryliques à base de cuivre.

11. Utilisation de diphosphines de formule 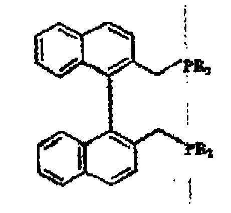 avec
R = 3,5 -C₆H₃F₂; -C₆F₅ ; 2,4,5 -C₆H₂F₃, 3,5-C₆H₃(CF₃)₂, 3,5 -C₆H₃(CH₃)₂, ou 3,4,5-C₆H₂F₃
en tant que rhodium-ligands dans un procédé d'hydroformylation d'oléfines comprenant 3 à 16 atomes de C sous catalyse par rhodium, la sélectivité n/i des aldéhydes obtenus étant supérieure à 85 : 15.

12. Utilisation selon la revendication 11,
**caractérisée en ce qu'**
une oléfine, du butène, un produit de raffinage I, un produit de raffinage II, du pentène et/ou de l'octène sont utilisés respectivement comme isomères purs ou mélange d'isomères.
